# EUROPÄISCHE PATENTANMELDUNG

(11) **EP 0 529 396 A1**
(43) Veröffentlichungstag der Anmeldung: **03.03.1993**
(21) Anmeldenummer: 92113641.2
(22) Anmeldetag: 11.08.1992
(51) Int. Cl.: A61K 9/20, A61K 9/16

(54) **Galenisch Matrix**

(30) Priorität: 22.08.1991 DE 4127665
(71) Anmelder: Beiersdorf Aktiengesellschaft, D-20245 Hamburg (DE)
(72) Erfinder: Heinze, Friedrich, Dr., W-2000 Hamburg 60 (DE); Wischniewski, Martin, Dr., W-3016Seelze 1 (DE); Brinckman, Silke, W-2080 Pinneberg (DE)

(57) **Zusammenfassung**

Galenische Matrices, enthaltend Zusammensetzungen aus
(a) Mikrokristalliner Cellulose,
(b) hochdispersem Siliciumdioxid,
(c) wenigstens einen lipophilen Bestandteil, gewählt aus der Gruppe der Öle, Fette und Wachse sowie
(d) gegebenenfalls Acrylatpolymeren und/oder -copolymeren,
sowie gegebenenfalls einen oder mehrere kosmetische oder pharmazeutische Wirkstoffe.

## Beschreibung

Die vorliegende Erfindung betrifft eine kosmetische und pharmazeutische Matrix, bevorzugt eine feinpartikuläre Wirkstoffträgermatrix. Insbesondere betrifft die Erfindung eine Wirkstoffträgermatrix zur gezielten und/oder verzögerten Freisetzung kosmetischer oder pharmazeutischer Wirkstoffe. Ferner betrifft die Erfindung eine Trägermatrix, mit Hilfe deren es möglich ist, unerwünschte Stoffe, beispielsweise überschüssige Körperexsudate vom Körper zu entfernen. Weiterhin betrifft die Erfindung Verfahren zur Herstellung einer Wirkstoffträgermatrix sowie deren Verwendung für kosmetische und pharmazeutische Zwecke.

Die Erfindung betrifft die topische Applikation.

Wirkstoffträgersysteme für kosmetische oder pharmazeutische Verwendungen sind an sich gut bekannt und weit verbreitet. Beispiele für partikuläre Systeme sind Mikrokapseln, Nanokapseln und Liposomen bzw. Niosomen. Das einfachste Wirkstoffträgersystem ist die kosmetische oder pharmazeutische Emulsion, bei welcher ein Wirkstoff in die dispergierte Phase eingearbeitet ist.

Ein grobes Unterscheidungsraster für diese Darreichungsformen ist ihre Größe. Die Größe der Mikrokapseln, wie auch die der dispergierten Emulsionströpfchen, liegt im Mikrometerbereich. Die Größe der Nanokapseln liegt im Nanometerbereich. Liposomen und Niosomen nehmen Größen einiger Nanometer bis einiger Mikrometer ein. Niosomen stellen Varianten der Liposomen dar, welche sich durch eine besondere Membranzusammensetzung auszeichnen.

Wirkstoffträger werden einesteils eingesetzt, um den oder die Wirkstoffe vor Umwelteinflüssen wie Oxidation oder Licht zu schützen. Ein anderer Bestimmungszweck kann sein, mit Hilfe des betreffenden Trägersystems den oder die Wirkstoffe gezielt am Anwendungsort freizusetzen. So wird beispielsweise angestrebt, mit Hilfe von kosmetischen Zubereitungen auf der Basis von Liposomen Wirkstoffe nicht auf der Haut verweilen zu lassen, sondern sie durch Diffusion durch die oberste in die tieferen Hautschichten zu transportieren. Dort soll sich die Wandung der Liposomen auflösen und die Wirkstoffe freisetzen. Würde der Wirkstoff ohne ein solches System auf die Haut aufgetragen, so würde der Wirkstoff nutzlos oder doch größtenteils ungenutzt auf der Haut verbleiben, oder er würde nicht optimal oder exakt am eigentlichen Wirkort zur Wirkung gelangen.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen, irritativen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern. Dies ist allerdings oft nur durch Einsatz kosmetisch aktiver Wirkstoffe zu bewerkstelligen. Ziel der Entwicklung kosmetischer Zubereitungen ist es dann, die Wirkstoffe stabil in die Formulierungen einzuarbeiten und dort über längere Zeit stabil aufrechtzuerhalten.

Es kann auch im Sinne der Hautpflege sein, überschüssige Hautfette, Schweiß und deren Zersetzungsprodukte von der Haut zu entfernen. Im allgemeinen lösen Seifen und synthetische Detergentien diese Aufgabe. Da diese jedoch meist des Guten zuviel tun, wird Dusch- und Badepräparaten oft ein rückfettendes Agens zugesetzt.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Die Zubereitungen des Standes der Technik weisen jedoch einige Nachteile auf: Durch viele der betreffenden Darreichungsformen die chemische Stabilisierung mancher oxidationsempfindlichen, lichtempfindlichen oder hydrolyseempfindlichen Stoffen nicht gewährleistet. Sollen pharmazeutische Wirkstoffe zur Anwendung gebracht werden, ist dieser Nachteil natürlich besonders bedenklich. So ist im Falle von in Emulsionen gelösten Wirkstoffen mit chemischen Umwandlungen der Wirkstoffe oder Reaktionen verschiedener Wirkstoffe untereinander oder Reaktionen der Wirkstoffe mit anderen Stoffen, z.B: Hilfs- oder Zusatzstoffen zu rechnen.

Bei kosmetischer Anwendung ist zu beklagen, daß insbesondere die Fixierung leichtflüchtiger Substanzen, z.B. etherischer Öle, Aromen, Parfums meist unzureichend ist. Aber auch der Nachteil, daß auch viele kosmetische Wirkstoffe instabil sind und gegen Denaturierung geschützt werden müssen, läßt sich durch herkömmliche Wirkstoffträgersysteme nur in begrenztem Maße bewerkstelligen.

Auch die Freisetzungscharakteristiken der Zubereitungen des Standes der Technik sind häufig ungenügend. So kann es geschehen, daß manche Matrices einen Wirkstoff in einem einzigen Schub abgeben, andere wiederum geben den eingearbeiteten Stoff so langsam ab, daß eine kosmetische oder therapeutische Wirkung nicht zustandekommt.

Aus der Firmenschrift von Dow Corning "Offene Fragen zur Verarbeitung von Polytrap R", unter dem Kapitel "Acrylates Copolymer: A Technique for Entrapping Cosmetic Actives" von W.L.Klein und A.J.DiSapio, Dow Corning, Montgomery, New York, sind Zusammensetzungen bekannt, in welchen eine Acrylatmatrix als Trägermatrix für Lidschatten und Rouge sowie als Absorptionsmatrix eines Hautfettadsorbers dient.

Diese Zusammensetzungen haben jedoch den Nachteil, daß die Matrices beim Auftragen auf die Haut zu leicht zerbröseln. Die erwünschte gleichmäßige Freigabe eines Wirkstoffes kann auf diese Weise nicht bewirkt werden. Angestrebt ist vielmehr, daß die Matrix allmählich durch Reibung an Substanz verliert und dabei, indem sie sich in immer kleinere Fragmente zerteilt, den Wirkstoff in die Haut einmassiert. Dies bedeutet, daß die Matrix nach dem Erosionsprinzip den Wirkstoff verzögert freisetzt.

Angestrebt ist ferner, daß die Matrix dabei als Festkörper erhalten bleibt und nicht in den gelösten Zustand übergeht.

Der Stand der Technik weist hier keine brauchbaren Matrices auf. Alle bekannten feinpartikulären Trägersysteme weisen mindestens einen der folgenden Nachteile auf:
Die Partikel sind schlecht verteilbar, sie sind porös und überdies in den meisten Fallen von sehr unregelmäßiger Form und keineswegs rund.

Die Partikelgrößenverteilung ist äußerst inhomogen. Dadurch ist eine gezielte Steuerbarkeit der Wirkstofffreisetzung nicht gewährleistet.

Die Partikel sind nur im Labormaßstab erhältlich, aber nicht großtechnisch herstellbar.

Die Partikel sind von mangelhafter mechanischer Resistenz. Daher sind sie nur in ungenügender Weise verteilbar und/oder lagerstabil. Ihre in den Endformulierungen eigentlich angestrebten Eigenschaften sind somit nicht erreichbar.

Aufgabe der vorliegenden Erfindung war also, Mittel zur Verfügung zu stellen, welche nicht mit den Mängeln des Standes der Technik behaftet sind. Insbesondere sollten Trägermatrices zur Verfügung gestellt werden, welche nicht nur über günstige Freigabecharakteristika für kosmetische und pharmazeutische Wirkstoffe verfügen, sondern überdies als Absorptionsmaterial zum Abtransport unerwünschter Stoffe, beispielsweise überschüssiger Körperexsudate geeignet sind.

Es hat sich überraschend herausgestellt, und darin liegt die Lösung der Aufgabe begründet, daß Zusammensetzungen aus
(a) Mikrokristalliner Cellulose,
(b) hochdispersem Siliciumdioxid,
(c) wenigstens einen lipophilen Bestandteil, gewählt aus der Gruppe der Öle, Fette und Wachse
(d) gegebenenfalls Acrylatpolymeren und/oder -copolymeren
hervorragende Galenische Matrices darstellen, die die Probleme des Standes der Technik lösen und den Zubereitungen des Standes der Technik Technik überlegen sind.

Im Zusammenhange mit der vorliegenden Erfindung bedeutet der Begriff "Galenische Matrix" einen Festkörper, der in der Kosmetik und/oder der Pharmazie als Trägermaterial dient. Dabei ist in den Begriff eingeschlossen, daß die Matrix als Träger für Wirkstoffe dienen kann wie auch als Adsorptionsmittel oder Absorptionsmittel für unerwünschte Stoffe.

Insbesondere hat die vorliegende Erfindung den Vorteil, daß es mit ihrer Hilfe möglich ist, extrudierbare Massen zu gewinnen, welche auf einfache Weise in handelsüblichen Extrudern verarbeitet werden können, ohne daß an den zu wählenden Temperaturbereich, die erforderlichen Maschinenteile oder sonstige Parameter besondere Anforderungen gestellt würden.

Darüberhinaus ist es ein besonderer Vorteil, daß erfindungsgemäß thermolabile Wirkstoffe gegebenenfalls erst bei niedrigeren Temperaturen, beispielsweise Raumtemperatur zugesetzt werden und so besonders schonend weiterverarbeitet werden können.

Vorteilhaft und überraschend ist ferner, daß die erfindungsgemäßen galenischen Matrices nach dem Extrusionsvorgang durch übliche Techniken zu kugelförmigen oder wenigstens annäherungsweise kugelförmigen Objekten geformt werden, und diese aufgrund ihrer hohen mechanischen Resistenz problemlos in übliche kosmetische oder pharmazeutische Grundlagen eingearbeitet werden können. Vielmehr war aber in Kenntnis des Standes der Technik anzunehmen, daß aufgrund der Zusammensetzung, insbesondere des Siliciumdioxidanteils, die Extrudate beim Versuch der Sphäronisierung zerbröckeln oder bei Zusatz von Lipidanteilen verkleben oder agglomerieren würden.

Die Mikrokristalline Cellulose wird bevorzugt gewählt aus der Gruppe der Cellulosearten, die ein Molgewicht von etwa 20000 bis 80000, insbesondere 30000 - 50000 besitzen. Cellulosearten die besonders vorteilhaft sind, zeichnen sich durch Partikelgrößen von etwa 1 - 100 µm, insbesondere aber 10 - 50 µm aus.

Handelsprodukte sind beispielsweise unter der Handelsbezeichnung Avicel^{R} erhältlich.

Das hochdisperse Siliciumdioxid wird bevorzugt gewählt aus der Gruppe der hochreinen, röntgenamorphen Siliciumdioxidsorten, insbesondere solchen, die durch Hydrolyse von SiCl₄ in einer Knallgasflamme erzeugt werden können, besonders bevorzugt aus der Gruppe der Produkte, welche die Handelsbezeichnung Aerosil^{R} tragen.

Die Polyacrylate werden vorteilhaft gewählt aus den Polymeren der Gruppe der Struktureinheit 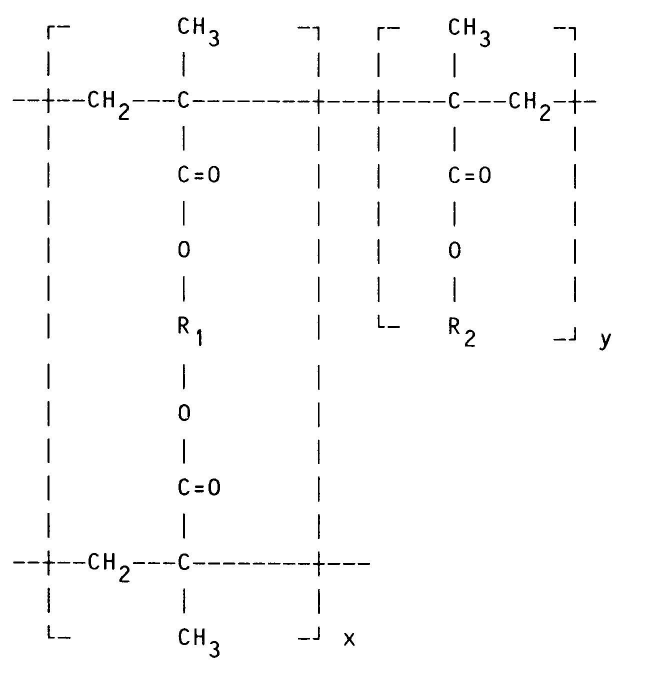
R₁ stellte dabei einen Niedrigalkylenrest dar, R₂ einen Niedrigalkylrest. Das Verhältnis von x : y zueinander ist vorteilhaft aus dem Bereich von etwa 1 : 5 bis 5 : 1 zu wählen.

Vorteilhaft ist insbesondere, die Acrylatpolymere aus der Gruppe der mit der Handelsbezeichnung "Polytrap" versehenen Polymere zu wählen. Von den letzteren ist das Produkt Polytrap Q5-6603 das Bevorzugte.

Vorteilhafte Zubereitungen betreffen Zusammensetzungen aus

| | | |
|---|---|---|
| (a) | 1 - 40 Gew.-% | mikrokristalliner Cellulose |
| (b) | 5 - 95 Gew.-% | eines lipophilen Bestandteils, gewählt aus der Gruppe der Fette, Öle und Wachse |
| (c) | 1 - 45 Gew.-% | hochdispersen Siliciumdioxids sowie |
| (d) | 0 - 35 Gew.-% | eines Acrylat-Polymers, sowie |

gegebenenfalls einen oder mehrere kosmetische oder pharmazeutische Wirkstoffe und gegebenenfalls Hilfs- und/oder Zusatzstoffe.

Bevorzugt enthalten die erfindungsgemäßen Zusammensetzungen

| | | |
|---|---|---|
| (a) | 1 - 20 Gew.-% | mikrokristalliner Cellulose |
| (b) | 20 - 95 Gew.-% | eines lipophilen Bestandteils, gewählt aus der Gruppe der Fette, Öle und Wachse |
| (c) | 1 - 45 Gew.-% | hochdispersen Siliciumdioxids sowie |
| (d) | 1 - 20 Gew.-% | eines Acrylat-Polymers, sowie |

gegebenenfalls einen oder mehrere kosmetische oder pharmazeutische Wirkstoffe und gegebenenfalls Hilfs- und/oder Zusatzstoffe.

Ganz besonders vorteilhaft sind Zusammensetzungen, enthaltend

| | | |
|---|---|---|
| (a) | 4 - 15 Gew.-% | mikrokristalliner Cellulose |
| (b) | 50 - 95 Gew.-% | eines lipophilen Bestandteils, gewählt aus der Gruppe der Fette, Öle und Wachse |
| (c) | 1 - 10 Gew.-% | hochdispersen Siliciumdioxids sowie |
| (d) | 5 - 15 Gew.-% | eines Acrylat-Polymers, sowie |

gegebenenfalls einen oder mehrere kosmetische oder pharmazeutische Wirkstoffe und gegebenenfalls Hilfs- und/oder Zusatzstoffe.

Die lipophilen Wirkstoffe können dabei vorteilhaft in die Gruppe (b) einbezogen und einberechnet werden.

Die Öle, Fette und Wachse können aus allen in Kosmetik und Pharmakologie üblichen Komponenten gewählt werden, als da sind Paraffinöle und -wachse, pflanzliche und tierische Öle, Fette und Wachse, z.B. Hanfwachs, Flachswachs, Bienenwachs und dergleichen, Silikonöle, synthetische Wachse, z.B. Cetylpalmitat, Cetylstearat und dergleichen, Mono,- Di- und Triglyceride, Fettsäuren, Fettalkohole, Montanwachse und dergleichen mehr.

Die Wirkstoffe können sehr vorteilhaft gewählt werden aus der Gruppe der lipophilen Wirkstoffe, insbesondere aus folgender Gruppe:
Acetylsalicylsäure, Atropin, Azulen, Hydrocortison und dessen Derivaten, z.B. Hydrocortison-17-valerat, Vitamine, z.B. Vitamine der B- und D-Reihe, sehr günstig das Vitamin B₁, das Vitamin B₁₂, das Vitamin D₃, aber auch die Vitamine A, E, K, Bisabolol, ungesättigte Fettsäuren, namentlich die essentiellen Fettsäuren (oft auch Vitamin F genannt), insbesondere die gamma-Linolensäure und Ölsäure, Chloramphenicol, Prostaglandine, Thymol, Campher, Extrakte oder andere Produkte pflanzlicher und tierischer Herkunft, z.B. Fischöle, Lebertran und so weiter.

Besonders vorteilhaft ist es auch, die Wirkstoffe aus der Gruppe der rückfettenden Substanzen zu wählen, beispielsweise, Eucerit und Neocerit.

Mit dieser Auflistung ist das Potential der vorliegenden Erfindung natürlich nicht erschöpft. Vielmehr kann der Fachmann ohne erfinderisches Dazutun weitere Wirkstoffe verwenden, ohne das Gebiet der vorliegenden Erfindung zu verlassen.

Bevorzugte Hilfs- und Zusatzstoffe sind beispielsweise Verdicker, Füllstoffe, Parfümöle, Farbstoffe, Emulgatoren, Wirkstoffe wie Vitamine oder Proteine, Lipidkomponenten, insbesondere Hautverwandte Lipide oder Lipide die denen der Haut ähneln, Trennmittel, pharmazeutische Wirkstoffe, Lichtschutzmittel, Stabilisatoren, Antioxidantien, Insektenrepellentien, Alkohol, Wasser, Salze, proteolytisch oder keratolytisch wirksame Substanzen usw.

Die erfindungsgemäßen Zusammensetzungen können vorteilhaft nach folgendem Verfahren hergestellt werden. Geräte, welche sich für diese Verfahren als sehr günstig herausgestellt haben sind Pelletierpressen von Schlüter und "Aeromatik"-Wirbelschichtapparaturen von Glatt .

Die Bestandteile (a) - (d), gegebenenfalls der oder die Wirkstoffe, sowie alle etwaigen Hilfs- und Zusatzstoffe, werden vereinigt und in einem temperierbaren Mischkneter bei Temperaturen zwischen 25°C und 60°C unter ständiger Agitation gehalten. Die auf diese Weise gewonnene Masse wird mit Hilfe einem geeigneten Hilfsmittel, sehr vorteilhaft einer temperierbaren Ringmatrizenpresse, zu zylinderförmigen Preßagglomeraten extrudiert. Diese Agglomerate werden, vorteilhaft in einer Wirbelschichtapparatur, auf ca 20°C - 30°C abgekühlt. Auch andere Kühlvorrichtungen sind gegebenenfalls von Vorteil.

Für thermisch weniger stabile Bestandteile kann das erfindungsgemäße Verfahren sehr günstig auf folgende Weise modifiziert werden:
Die Bestandteile (a) - (d) und alle etwaigen Hilfs- und Zusatzstoffe, werden vereinigt und in einem temperierbaren Mischkneter bei Temperaturen zwischen 25°C und 40°C unter ständiger Agitation gehalten. Die auf diese Weise gewonnene Masse wird abgekühlt und der oder die thermolabilen Wirkstoffe zugegeben und in einem schnellaufenden Mischkneter (z.B. einem Diosna- Mischer) homogen verteilt. Anschließend wird die auf diese Weise entstandene wirkstoffhaltige Matrix mit Hilfe eines geeigneten Hilfsmittels, sehr vorteilhaft einer temperierbaren Ringmatrizenpresse, zu zylinderförmigen Preßagglomeraten extrudiert. Diese Agglomerate werden, vorteilhaft in einer Wirbelschichtapparatur auf ca 20°C - 25°C abgekühlt.

Als Variante dieser beiden Verfahren ist das Folgende zu sehen:
Die Bestandteile (a) - (d), gegebenenfalls der oder die thermisch stabilen Wirkstoffe, sowie alle etwaigen Hilfs- und Zusatzstoffe, werden vereinigt und in einem temperierbaren Mischkneter bei Temperaturen zwischen 25°C und 60°C unter ständiger Agitation gehalten. Die auf diese Weise gewonnene Masse wird abgekühlt und der oder die thermolabilen Wirkstoffe zugegeben und in einem schnellaufenden Mischkneter (z.B. einem Diosna- Mischer) homogen verteilt. Anschließend wird die auf diese Weise entstandene wirkstoffhaltige Matrix mit Hilfe eines geeigneten Hilfsmittels, sehr vorteilhaft einer temperierbaren Ringmatrizenpresse, zu zylinderförmigen Preßagglomeraten extrudiert. Diese Agglomerate werden, vorteilhaft in einer Wirbelschichtapparatur, auf ca 20°C - 30°C abgekühlt. Auch andere Kühlvorrichtungen sind gegebenenfalls von Vorteil.

Die Preßagglomerate können der sogenannten Spheronisierung unterworfen werden, was bedeutet, daß sie nach an sich bekannten Verfahren in Kugelgestalt gebracht werden können. Sie werden bei diesem Vorgang vorteilhaft auf schnellaufenden Spheronizerscheiben durch intensives Rollieren während eines Zeitintervalls von typischerweise 5 - 15 Minuten zu kleinen Kugeln verarbeitet, die sehr günstig im Größenbereich zwischen 250 µm und 5 mm liegen können. Dem Fachmanne sind die dazu geeigneten Geräte unter der Bezeichnung Marumerizer oder Spheronizer bekannt.

Das erfindungsgemäße Verfahren erlaubt es erstmals, zu überwiegendem Teil lipophile, also in erster Linie überwiegend wachs-, fett- und/oder ölhaltige Massen mit Hilfe der an sich bekannten Extrudertechnologie zu spheronisierbaren Agglomeraten zu verarbeiten. Erstaunlich und für den Fachmann unvorhersehbar war, daß die Zusammensetzungen gemäß der vorliegenden Erfindung in den entsprechenden Temperaturintervallen genügend große plastische Verformungseigenschaften besitzen, um sie erfolgreich der Extrusion unterwerfen zu können. Erstaunlich war ferner, daß die Extrudate zudem erfolgreich durch die an sich bekannten Verfahren der Spheronisierung zu kleinen Kugeln verarbeitet werden können. Dabei sind die Partikeldurchmesser der Kugeln mit großer Exaktheit reproduzierbar. Es ist ferner möglich und vorteilhaft, aus den erfindungsgemäßen Matrices Partikel mit einem sehr eng begrenzten Partikelgrößenintervall herzustellen. Außerdem kann die Massenausbeute an solchen Partikeln mehr als 95 Gew.-% betragen.

Ein erheblicher Vorteil des möglichen, eng begrenzten Partikelgrößenintervalles ist, daß Formulierungen, enthaltend die erfindungsgemäßen galenischen Matrices eingearbeitete Wirkstoffe gezielt und/oder kontrolliert freisetzen können, obwohl schon die Matrices an sich über hervorragende Eigenschaften dieser Art verfügen.

Ein weiterer überragender Vorteil der vorliegenden Erfindung ist, daß die Extrudate bzw. die durch Sphäronisierung gewonnenen Kugeln die Stabilität eines eingearbeiteten Wirkstoffes drastisch erhöhen und auch die Eigenschaften besitzen, einen Wirkstoff der kontrollierten, bzw. verzögerten Freisetzung zur Verfügung zu stellen.

Es ist möglich und vorteilhaft, die erfindungsgemäßen galenischen Matrices in die üblichen Zubereiteungen des Standes der Technik einzuarbeiten, beispielsweise in hydrophile Gele, Trägersysteme, Lipogele, Salben, Crèmes, einfache Emulsionen (der Typen W/O und O/W), multiple Emulsionen (der Typen W/O/W, O/W/O und so weiter), Mischemulsionen, Suspensionen und Lösungen.

Als besonders vorteilhafte Verkörperung der vorliegenden Erfindung wird angesehen, die erfindungsgemäßen Matrices in emulgatorfreie Systeme einzuarbeiten, also insbesondere in Gele oder Lösungen.

Für Hilfs-und Zusatzstoffe sind auch hier alle dem Fachmanne an sich bekannten Stoffe dieser Stoffgattungen möglich und vorteilhaft zu verwenden.

Es ist weiterhin von Vorteil, die erfindungsgemäßen Matrices mit den in der Emulsionstechnologie üblichen Mischersystemen den endgültigen Formulierungen einzuverleiben.

Die vorliegende Erfindung gestattet, lipophile Hilfs- und Wirkstoffe emulgatorfrei in der Matrix und, wenn erwünscht, in der jeweiligen Zubereitung zu halten, beispielsweise in Gelen oder Lösungen, so daß es möglich ist, in diesen Systemen das hautirritative und/oder allergene Potential dieser Substanzklasse zu vermeiden. Die Erfindung stellt dabei eine Kompartimentierung vom Typus fest/fest, bzw. fest/halbfest im pharmazeutisch- technologischen Sinne, dar. Damit wird ein stabiles Zweiphasensystem geschaffen, welches die Trennung chemisch inkompatibler Substanzen in ein und demselben Präparat erlaubt und dadurch zusätzliche Stabilität schafft. Die damit verbundene Immobilisierung der Wirkstoffe verringert zudem das Risiko möglicher unerwünschter Reaktionen mit Stoffen der umgebenden flüssigen Phase (z.B. in Gelen, Emulsionen, Lipogelen usw.).

Durch den vesikulären Verteilungszustand in den Zubereitungen ist eine Verringerung der reaktiven Oberfläche des oder der Wirkstoffe erreichbar.

Auch die endgültigen kosmetischen und pharmazeutischen Zubereitungen zeichnen sich durch äußerst vorteilhafte Wirkung aus. Bei ihnen zeigt sich insbesondere die hohe Befähigung zur kontrollierten und gesteuerten Wirkstofffreisetzung nach topischer Applikation.

Sind die Zusammensetzungen gemäß der vorliegenden Erfindung kosmetischer Natur, so sind folgende Verwendungen besonders bevorzugt:
Scrub-Produkte, Kosmetikgele, Gesichts- und Körperpflegezubereitungen, Peelingprodukte, Reinigungspräparate, emulgatorfreie Zubereitungen, Sonnenschutzmittel, Insektenrepellentien, Massagegele, Sportgele, sowie alle kosmetischen Zubereitungen, welche über einen gewissen Zeitraum auf oder in die Haut massiert werden.

Die Formulierung der jeweiligen kosmetischen Basis dieser Produkte ist dem Fachmanne an sich bekannt.

Die folgenden Beispiele dienen dazu das Wesen der vorliegenden Erfindung zu erläutern, ohne daß aber beabsichtigt ist, die Erfindung auf diese Beispiele zu beschränken. Vielmehr kann der Fachmann eine Fülle von Variationen vornehmen, die seinem allgemeinen Fachwissen entspringen, die den Boden der vorliegenden Erfindung aber nicht verlassen. Die Mengenangaben der Beispiele bedeuten Gewichts-%, sofern nicht ausdrücklich anders gekennzeichnet.

### Beispiel 1

### Kugelförmige Lipidträgerpartikel, beispielsweise für Kosmetikgel

| | |
|---|---|
| Mikrokristalline Cellulose | 10,00 |
| Jojobaöl | 15,00 |
| Cetylstearylalkohol | 38,50 |
| Aerosil R972 | 4,00 |
| Polytrap 6603 | 12,00 |
| alpha-Tocopherol | 10,00 |
| Bisabolol | 10,00 |
| Glycerin | 1,20 |
| Farbpigmente | 0,30 |

### Beispiel 2

### Kugelförmige Lipidträgerpartikel, beispielsweise für Kosmetikgel

| | |
|---|---|
| Mikrokristalline Cellulose | 15,00 |
| Jojobaöl | 15,00 |
| Cetylstearylalkohol | 38,50 |
| Aerosil R972 | 10,00 |
| alpha-Tocopherol | 10,00 |
| Bisabolol | 10,00 |
| Glycerin | 1,20 |
| Farbpigmente | 0,30 |

Die Bestandteile gemäß den Beispielen 1 und 2 werden nach folgendem Verfahren aufbereitet:
Alle Bestandteile werden vereinigt und in einem temperierbaren Mischkneter bei einer Temperatur von 35°C und 40°C unter ständiger Agitation gehalten. Die auf diese Weise gewonnene Masse wird in einer temperierbaren Ringmatrizenpresse der Firma Schlüter zu zylinderförmigen Preßagglomeraten extrudiert. Diese Agglomerate werden in einer Wirbelschichtapparatur der Firma Glatt auf ca 25°C abgekühlt. Die Preßagglomerate werden auf schnellaufenden Spheronizerscheiben durch intensives Rollieren während eines Zeitintervalls von 10 Minuten zu kleinen Kugeln verarbeitet, die etwa 1 mm Durchmesser aufweisen.

### Beispiel 3

### Linolensäureträger für Dermatherapeutikum

| | |
|---|---|
| gamma-Linolensäure | 10,00 |
| Paraffinum perliq. | 25,00 |
| Cetylpalmitat | 43,50 |
| Aerosil 200 | 10,00 |
| Aerosil R972 | 3,00 |
| Mikrokristalline Cellulose | 10,00 |
| Glycerin | 2,50 |

Die Bestandteile gemäß Beispielen 3 werden nach folgendem Verfahren aufbereitet:
Alle Bestandteile, mit Ausnahme der gamma-Linolensäure, werden vereinigt und in einem temperierbaren Mischkneter bei einer Temperatur von 35°C und 40°C unter ständiger Agitation gehalten. Nach Abkühlen auf ca. 25°C wird die gamma-Linolensäure zugegeben und im Mischkneter homogen in der Masse verteilt. Die auf diese Weise gewonnene Masse wird in einer temperierbaren Ringmatrizenpresse der Firma Schlüter zu zylinderförmigen Preßagglomeraten extrudiert. Diese Agglomerate werden in einer Wirbelschichtapparatur der Firma Glatt auf ca 25°C abgekühlt.

### Versuch 1

Kugelförmige Lipidträgerpartikel, die aus einer Zusammensetzung gemäß Beispiel 1 nach dem oben beschriebenen Verfahren gewonnen wurden, wurden anhand des folgenden Belastungsversuches untersucht:
Auf einer quadratischen Glasplatte der Kantenlänge 20 cm wurden 200 mg kugelförmiger Lipidträgerpartikel verteilt. Eine ausgewogene Glasplatte der gleichen Größe wurde aufgelegt und mit einem Glasgefäß belastet, in welches in 100-ml-Schritten Wasser eingefüllt wurde. Zwischen den Auffüllschritten wurde der Auffüllvorgang 30 Sekunden lang unterbrochen. Es wurden 5 Experimente auf diese Weise durchgeführt.

Das Gesamtbelastungsgewicht bei Zerdrücken der ersten Partikel (T₁) sowie das Gesamtbelastungsgewicht bei Zerdrücken der letzten Partikel wurde ermittelt.

| Experiment | T₁ | T₂ |
|---|---|---|
| 1 | 1625 g | 3485 g |
| 2 | 1630 g | 4030 g |
| 3 | 2900 g | 3500 g |
| 4 | 1450 g | 3050 g |
| 5 | 2790 g | >5000 g |

### Versuch 2

Kugelförmige Lipidträgerpartikel, die aus einer Zusammensetzung gemäß Beispiel 2 nach dem oben beschriebenen Verfahren gewonnen wurden, wurden in Analogie zu Versuch 1 untersucht:

Das Gesamtbelastungsgewicht bei Zerdrücken der ersten Partikel (T₁) sowie das Gesamtbelastungsgewicht bei Zerdrücken der letzten Partikel wurde ermittelt. Es wurden 5 Experimente auf diese Weise durchgeführt.

| Experiment | T₁ | T₂ |
|---|---|---|
| 6 | 1555 g | 1200 g |
| 7 | 1580 g | 3800 g |
| 8 | 2790 g | 3400 g |
| 9 | 1400 g | 2890 g |
| 10 | 2700 g | 4890 g |

Ersichtlich ist, daß die an sich schon hohe Stabilität der Zubereitung aus mikrokristalliner Cellulose und hochdispersem Siliciumdioxid (Versuch 2) noch durch einen zusätzlichen Gehalt an Acrylatpolymeren/copolymeren gesteigert werden kann.

### Beispiel 4

### Hautpflegegel mit kugelförmigen Lipidträgerpartikeln nach Beispiel 2

| | |
|---|---|
| Polyacrylsäure (Carbopol 940) | 0,50 |
| NaOH - Lösung (5 %) | 3,00 |
| Glycerin (85 %) | 10,00 |
| Partikel/Beispiel 2 | 3,00 |
| Sorbinsäure | 0,10 |
| Wasser VES | ad 100,00 |

## Patentansprüche

1. Galenische Matrices, enthaltend Zusammensetzungen aus
(a) Mikrokristalliner Cellulose,
(b) hochdispersem Siliciumdioxid,
(c) wenigstens einen lipophilen Bestandteil, gewählt aus der Gruppe der Öle, Fette und Wachse sowie
(d) gegebenenfalls Acrylatpolymeren und/oder -copolymeren,
sowie gegebenenfalls einen oder mehrere kosmetische oder pharmazeutische Wirkstoffe.

2. Galenische Matrices nach Anspruch 1, enthaltend Zusammensetzungen aus
| | | |
|---|---|---|
| (a) | 1 - 40 Gew.-% | mikrokristalliner Cellulose |
| (b) | 5 - 95 Gew.-% | eines lipophilen Bestandteils, gewählt aus der Gruppe der Fette, Öle und Wachse |
| (c) | 1 - 45 Gew.-% | hochdispersen Siliciumdioxids sowie |
| (d) | 0 - 35 Gew.-% | eines Acrylat-Polymers |
sowie gegebenenfalls einen oder mehrere kosmetische oder pharmazeutische Wirkstoffe und gegebenenfalls Hilfs- und/oder Zusatzstoffe.

3. Galenische Matrices nach Anspruch 1, enthaltend Zusammensetzungen aus
| | | |
|---|---|---|
| (a) | 1 - 20 Gew.-% | mikrokristalliner Cellulose |
| (b) | 20 - 95 Gew.-% | eines lipophilen Bestandteils, gewählt aus der Gruppe der Fette, Öle und Wachse |
| (c) | 1 - 45 Gew.-% | hochdispersen Siliciumdioxids sowie |
| (d) | 1 - 20 Gew.-% | eines Acrylat-Polymers. |
sowie gegebenenfalls einen oder mehrere kosmetische oder pharmazeutische Wirkstoffe und gegebenenfalls Hilfs- und/oder Zusatzstoffe.

4. Galenische Matrices nach Anspruch 1, enthaltend Zusammensetzungen aus
| | | |
|---|---|---|
| (a) | 4 - 15 Gew.-% | mikrokristalliner Cellulose |
| (b) | 50 - 95 Gew.-% | eines lipophilen Bestandteils, gewählt aus der Gruppe der Fette, Öle und Wachse |
| (d) | 1 - 10 Gew.-% | hochdispersen Siliciumdioxids sowie |
| (c) | 5 - 15 Gew.-% | eines Acrylat-Polymers. |
sowie gegebenenfalls einen oder mehrere kosmetische oder pharmazeutische Wirkstoffe und gegebenenfalls Hilfs- und/oder Zusatzstoffe.

5. Galenische Matrices nach einem der Ansprüche 1 - 4, dadurch gekennzeichnet, daß die Öle, Fette und Wachse aus der Gruppe der Paraffinöle und -wachse, pflanzliche und tierische Öle, Fette und Wachse, z.B. Hanfwachs, Flachswachs, Bienenwachs und dergleichen, Silikonöle, synthetische Wachse, z.B. Cetylpalmitat, Cetylstearat und dergleichen, Mono,- Di- und Triglyceride, Fettsäuren, Fettalkohole, Montanwachse gewählt werden.

6. Galenische Matrices nach einem der Ansprüche 1 - 5, dadurch gekennzeichnet, daß sie in kosmetische oder pharmazeutische Zubereitungen, beispielsweise in hydrophile Trägersysteme, Lipogele, Salben, Crèmes, einfache Emulsionen (der Typen W/O und O/W), multiple Emulsionen (der Typen W/O/W, O/W/O und so weiter), Suspensionen und Lösungen eingearbeitet sind.

7. Verwendung von Galenischen Matrices nach einem der Ansprüche 1 - 5 in einer pharmazeutischen Zubereitungen sowie kosmetischen Zubereitungen, insbesondere kosmetischen Zubereitungen gewählt aus der folgenden Gruppe:
Scrub-Produkte, Kosmetikgele, Gesichts- und Körperpflegezubereitungen, Peelingprodukte, Reinigungspräparate, emulgatorfreie Zubereitungen, Sonnenschutzmittel, Insektenrepellentien, Massagegele, Sportgele, sowie alle kosmetischen Zubereitungen, welche über einen gewissen Zeitraum auf oder in die Haut massiert werden.

8. Verfahren zur Herstellung Galenischer Matrices nach einem der Ansprüche 1 - 6, dadurch gekennzeichnet, daß
die Bestandteile (a) - (d), also
(a) Mikrokristalliner Cellulose,
(b) hochdispersem Siliciumdioxid,
(c) wenigstens einen lipophilen Bestandteil, gewählt aus der Gruppe der Öle, Fette und Wachse sowie
(d) gegebenenfalls Acrylatpolymeren und/oder -copolymeren und gegebenenfalls der oder die thermisch stabilen Wirkstoffe, sowie alle etwaigen Hilfs- und Zusatzstoffe, vereinigt und in einem temperierbaren Mischkneter bei Temperaturen zwischen 25°C und 60°C unter ständiger Agitation gehalten werden, dann, wenn thormolabile Wirkstoffe eingearbeitet werden sollen, nach Abkühlung, die thermolabilen Wirkstoffe zugegeben und mit der Masse homogenisiert werden, die auf diese Weise gewonnene Masse mit Hilfe einem geeigneten Hilfsmittel, sehr vorteilhaft einer temperierbaren Ringmatrizenpresse, zu zylinderförmigen Preßagglomeraten extrudiert wird, diese Agglomerate, vorteilhaft in einer Wirbelschichtapparatur, auf ca 20°C - 30°C abgekühlt werden, und die Preßagglomerate gegebenenfalls der sogenannten Sphäronisierung unterworfen werden, vorteilhaft dergestalt, daß sie auf schnellaufenden Spheronizerscheiben durch intensives Rollieren während eines Zeitintervalls von etwa 5 - 15 Minuten zu kleinen Kugeln verarbeitet werden.
